# EUROPEAN PATENT SPECIFICATION

(11) **EP 1 173 768 B1**
(45) Date of publication and mention of the grant of the patent: **25.10.2006**
(21) Application number: 00929470.3
(22) Date of filing: 02.05.2000
(51) Int. Cl.: G01N 33/68, A61K 38/18

(54) **Methods of diagnosing, prognosing, monitoring the progression or evaluating of a treatment of depression on basis of increased cerebrospinal fluid levels of neurotrophin 3**
Methode zur Diagnostizierung, Prognose oder Evaluation von Behandlung der Depressionen, basiert auf erhöhte Neurotrophin Concentrationen in zerebrospinale Flüssigkeit.
Procédés permettant le diagnostic, la prévison, l'évaluation de la progression ou du traitement de la dépression basés sur des quantités élevées de neurotrophin dans le liquide cérébrospinal.

(30) Priority: 03.05.1999 EP 99108723; 09.10.1999 EP 99120212
(43) Date of publication of application: 23.01.2002
(73) Proprietor: Evotec AG, 22525 Hamburg (DE)
(72) Inventor: NITSCH, Roger, 8126 Zumikon (CH); HOCK, Christoph, 8703 Erienbach (CH); OTTEN, Uwe, 4052 Basel (CH)
(74) Representative: Meyers, Hans-Wilhelm
(86) International application number: PCT/EP2000/003912
(87) International publication number: WO 2000/067032

(56) References cited:
- WO-A-91/03569
- WO-A-93/09798
- MURASE, K.; IGARASHI, K.; HAYASHI, K.: "Neurotrophin-3 (NT-3) levels in the developing rat nervous system and in human samples" CLINICA CHIMICA ACTA, vol. 227, no. 1-2, 1994, pages 23-36, XP000852953 cited in the application
- NARISAWA-SAITO M; WAKABAYASHI K; TSUJI S; TAKAHASHI H; NAWA H: "Regional specificity of alterations in NGF, BDNF and NT-3 levels in Alzheimer's disease" NEUROREPORT, vol. 7, no. 18, 25 November 1996 (1996-11-25), pages 2925-2928, XP000852945 cited in the application
- M BOTHWELL: "Functional interactions of neurotrophins and neurotrophin receptors" ANNUAL REVIEW OF NEUROSCIENCE, vol. 18, 1995, pages 223-253, XP000852678 cited in the application
- GILMORE JH, JARSKOG LF, LINDGREN JC, MCEVOY JP, XIAO H: "Neurotrophin-3 levels in the cerebrospinal fluid of patients with schizophrenia or medical illness" PSYCHIATRY RESEARCH, vol. 63, no. 1-2, 14 November 1997 (1997-11-14), pages 109-113, XP000852677 cited in the application
- E ARENAS, H PERSSON: "Neurotrophin-3 prevents the death of adult central noradrenergic neurons in vivo" NATURE, vol. 367, 27 January 1994 (1994-01-27), pages 368-371, XP002122262 cited in the application
- M A SMITH, S MAKINO, M ALTEMUS, D MICHELSON, S-K HONG, R KVETNANSKY, R M POST: "Stress and antidepressants differentially regulate neurotrophin 3 mRNA expression in the locus coeruleus" PROCEEDINGS OF THE NATIONAL ACADEMY OF SCIENCES OF USA, vol. 92, September 1995 (1995-09), pages 8788-8792, XP002122263 cited in the application
- EBADI, M.; BASHIR, R. M.; HEIDRICK, M. L.; HAMADA, F. M.; EL REFAEY, H.; HAMED, A.; HELAL, G.; BAXI, M. D. ET AL.: "Neurotrophins and their receptors in nerve injury and repair" NEUROCHEMISTRY INTERNATIONAL, vol. 30, no. 4/5, 1997, pages 347-374, XP000852951
- LEONARD BE: "The role of noradrenaline in depression: a review" JOURNAL OF PSYCHOPHARMACOLOGY, vol. 11, no. 4S, 1997, pages 39-47, XP000852762 cited in the application

## Description

The present invention relates to methods of diagnosing, prognosing, monitoring the progression or evaluating treatment of depression.

In the search for biochemical changes in patients with neuropsychiatric or neurodegenerative disorders analysis of cerebrospinal fluid (CSF) may be a useful method, since the CSF is continuous with the extracellular fluid of the brain. Therefore, a plurality of studies aiming at the analysis of the central nervous system (CNS) specific proteins in CSF were performed in order to find biochemical markers for neuronal and synaptic function and pathology in degenerative brain disorders.

Neurotrophin 3 (NT-3) is a member of the neurotrophin gene family which supports the survival, differentiation, maintenance, and repair of vertebrate neurons (Bothwell, Ann. Rev. Neurosci. 8, 223 - 253, 1995; Ebadi et al., Neurochemistry International 30 (4-5), 347-374, 1997). It was shown to prevent the death of adult central noradrenergic neurons of the locus coeruleus in a 6-hydroxydopamine lesion model (Arenas et al., Nature 367 (6461), 368 - 371, 1994), a neuronal population which is associated with the pathophysiology of major depression (Leonard, J. Psychopharmacol., 11 (4), 39 - 47, 1997). Alterations in adrenoreceptor density and function, and changes in adrenoreceptors associated with the pituitary-adrenal axis function strongly implicate a disorder in central noradrenergic transmission in major depression (for review see: Leonard, J. Psychopharma 11 (4), 39 - 47, 1997). This dysfunction might be related to the activity of tyrosine hydroxylase, the rate-limiting enzyme in the synthesis of catecholamines. Alternatively, impaired uptake or transport of a target-derived neurotrophic factor, such as NT-3, may contribute to central noradrenergic dysfunction.

The international patent application WO 91/03569 relates to NT-3 and its use in the diagnosis and/or treatment of neurologic disorders including peripheral neuropathies such as diabetic neuropathies, toxic and nutritional neuropathies, hereditary neuropathies and AIDS related neuropathies and degenerative diseases as Alzheimer's disease.

Murase et al. (Clinica Chimica Acta, 227(1-2), 23 - 36, 1994) developed an enzyme immunoassay system for NT-3, based on a biotin-streptadivin detection system. This system was applied to measure NT-3 levels in the developing rat nervous system as well as in normal subjects and patients with Alzheimer's disease.

Using two-site enzyme immunoassays, Narisawa-Saito et al. (Neuroreport 7(18), 2925 - 2928, 1996) measured inter alia NT-3 in the brain regions (motor cortex, dentate gyrus and entorhinal cortex) of patients with Alzheimer's disease and control individuals.

The international patent application WO93/09798 provides for therapeutic and diagnostic method based on human NT-3 expression, specifically the potential to treat Alzheimer's disease and Huntington's disease.

NT-3 was previously determined in the CSF of patients with a variety of neuropsychiatric conditions including hydrocephalus, meningitis, encephalitis, ventriculitis, brain tumors, and multiple sclerosis, with values ranging from 4.8 to 55.9 pg/ml (Gilmore et al., Psychiatry Res. 73(1-2) 109 - 113, 1997). In the report of Gilmore, NT-3 was not detectable in the CSF of patients with schizophrenia.

Up to date, studies on CSF levels of NT-3 in major depression in the elderly (DE) or in depressive populations of younger age have not yet been reported.

Despite its relevance for quality care initiatives, the field of psychiatry has little scientific knowledge regarding the course of current major depression when primary care patients with the disorder remain undetected. The risk that undetected patients develop suicidal ideation should not be underestimated. Therefore, methods of diagnosing depression, in particular major depression, as well as methods of treatment are urgently needed.

In one aspect, the invention features a method of claim 1.

In a further aspect, the invention features a method of claim 2.

In still a further aspect, the invention features a method of claim 3.

According to the invention, a body fluid sample is used, preferably cerebrospinal fluid sample. An increase of a level of neurotrophin 3 in said cerebrospinal fluid from said subject relative to said reference value representing a known health status indicates a diagnosis, or prognosis or increased risk of depression, in particular major depression, in said subject. In particular, a level of neurotrophin 3 ≥ 15 pg/ml in CSF indicates a diagnosis, or prognosis, or increased risk of depression, in particular major depression, in said subject. Specifically, a level of neurotrophin 3 in the range from 15 pg/ml to 50 pg/ml in CSF indicates a diagnosis, or prognosis, or increased risk of depression, in particular major depression, in said subject. The increases of CSF levels of NT-3 may reflect disturbances in the trophic support of specific neuronal populations, such as the central noradrenergic system in DE.

It is particularly preferred to compare a level, or an activity, or both said level and said activity, of neurotrophin 3 and/or of a transcription product of a gene coding for neurotrophin 3 in said sample with a level of at least one of said substances in a series of samples taken from said subject over a period of time. The said subject is a human being, receives a treatment prior to one or more of said sample gatherings. Neurotrohphin 3 and/or said transcription product can preferably be detected using an immunoassay and/or a binding assay.

In another aspect, the invention features the use of a kit as in claim 11. The kit may further comprise instructions for diagnosing, or prognosing depression, or determining increased risk of developing depression by
(i) detecting a level, or an activity, or both said level and said activity, of neurotrophin 3 and/or of a transcription product of a gene coding for neurotrophin 3 in a sample from said subject; and
(ii) diagnosing, or prognosing, or determining whether said subject is at increased risk of developing depression,
wherein a varied level, or activity, or both said level and said activity, of neurotrophin 3 and/or of a transcription product of a gene coding for neurotrophin 3 compared to a reference value representing a known health status;
or a level, or an activity, or both said level and said activity, of neurotrophin 3 and/or of a transcription product of a gene coding for neurotrophin 3 similar or equal to a reference value representing a known disease status indicates a diagnosis, or prognosis, or increased risk of developing depression.

In particular, a level of neurotrophin 3 ≥ 15 pg/ml in CSF indicates a diagnosis, or prognosis, or increased risk of depression, in particular major depression, in said subject. Specifically, a level of neurotrophin 3 in the range from 15 pg/ml to 50 pg/ml in CSF indicates a diagnosis, or prognosis, or increased risk of depression, in particular major depression, in said subject. Additionally, said kit preferably further comprises at least one reagent which selectively detects nerve growth factor or a transcription product of a gene coding for nerve growth factor. Combined testing of NT-3 and NGF is a valuable tool in the diagnosis, prognosis, or risk evaluation of the above mentioned diseases (see example 3 and table 1).

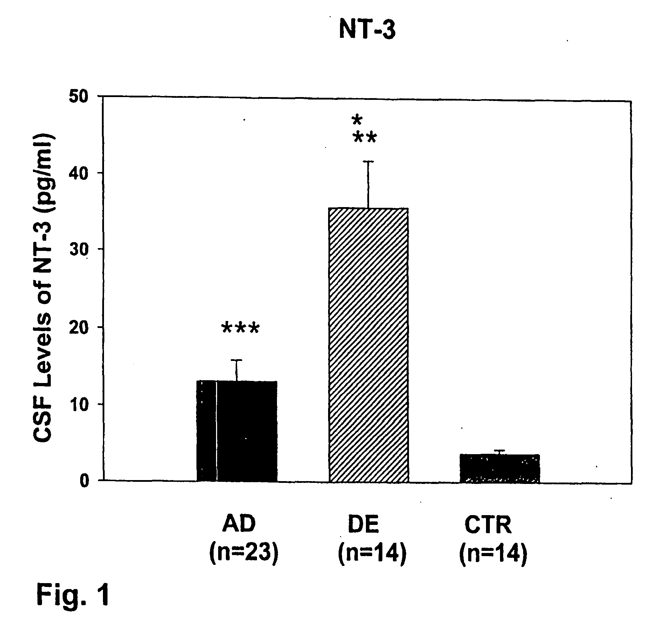

Figure 1 relates to example 1 and depicts that CSF levels of NT-3 were significantly elevated in the DE group, as compared to both the AD and the CTR groups. Levels (pg/ml) are given in mean ± SEM. Asterix (*, **, ***) indicate significance (p < 0.005), Mann-Whitney U Test. NT-3: * DE versus AD, p = 0.004; ** DE versus CTR, p < 0.001; *** AD versus CTR, p = 0.013. NT-3 concentrations in CSF of the DE group were 35.7 +/- 6.2 pg/ml (mean +/- SEM; range 2.3 to 87.00, n = 14), compared to 13.2 +/- 2.8 pg/ml in the AD group (range: 2.0 to 41.0, n = 23), and 3.7 +/- 0.5 pg/ml in the CTR group (range: 0.00 to 8.2, n = 14), respectively. In addition, CSF levels of NT-3 were significantly higher in the AD group as compared to the CTR group. There was no apparent correlation of CSF levels of NT-3 with age, duration of AD, MMS, NOSGER or MADRS scores.

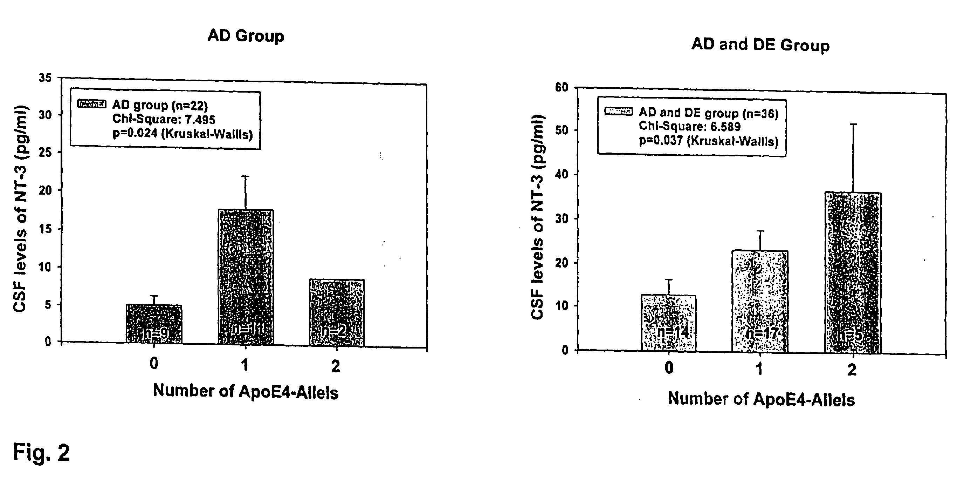

Figure 2 relates to example 1 and depicts CSF levels of NT-3 (pg/ml ± SEM) grouped for presence of ApoE4 alleles. Left panel: Alzheimer's disease group, n = 22, Kruskal-Wallis; χ² = 7.495, p = 0.024. Right panel: total sample of patients with AD and major depression (DE), n = 36, Kruskal-Wallis test: χ² = 6.589, p = 0.037. CSF levels of NT-3 increased with increasing ApoE4 allele frequency in the total sample of AD and DE patients and within the AD group. There was no apparent effect of the number of E4 alleles within the DE group (n = 14, Kruskal-Wallis test: χ² = 1.508, p = 0.471).

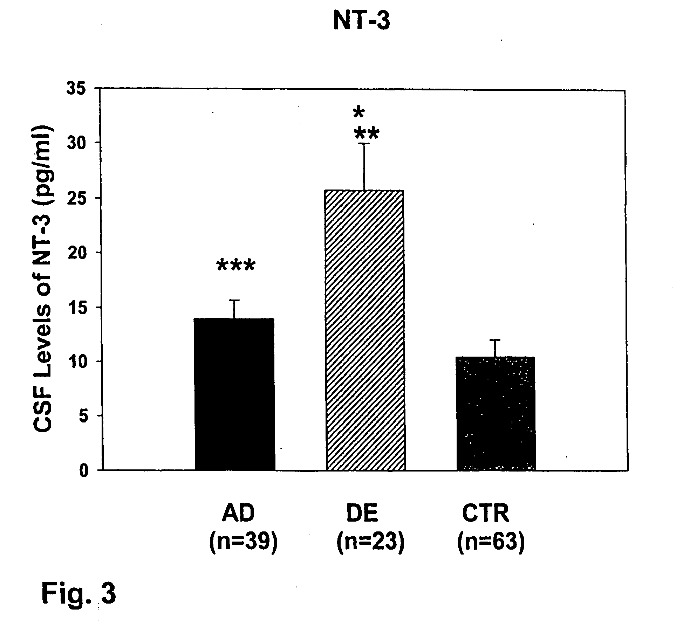

Figure 3 relates to example 2 and depicts NT-3 levels in the cerebrospinal fluid of patients with Alzheimer's disease (AD), major depression in the elderly (DE) and non-demented control subjects (CTR). Levels (pg/ml) are given in mean ± SEM. Asterix (*,**,***) indicate significance (p < 0.05), Mann-Whitney U Test. * DE versus AD, p = 0.005; ** DE versus CTR, p = 0:000; *** AD versus CTR, p = 0.010. CSF levels of NT-3 were significantly elevated in the DE group, as compared to both the AD and the CTR group. CSF levels of NT-3 were slightly, but significantly, elevated in the AD group, as compared to the CTR group. NT-3 concentrations in CSF of the DE group were 25.8 +/- 4.3 pg/ml (mean +/- SEM, range: 0.0 to 87.0, n = 23), compared to 14.0 +/- 1.6 pg/ml in the AD group (range: 0.0 to 41.0, n = 39), and 10.5 +/- 1.6 pg/ml in the CTR group (range: 0.0 to 67.0, n = 63), respectively.

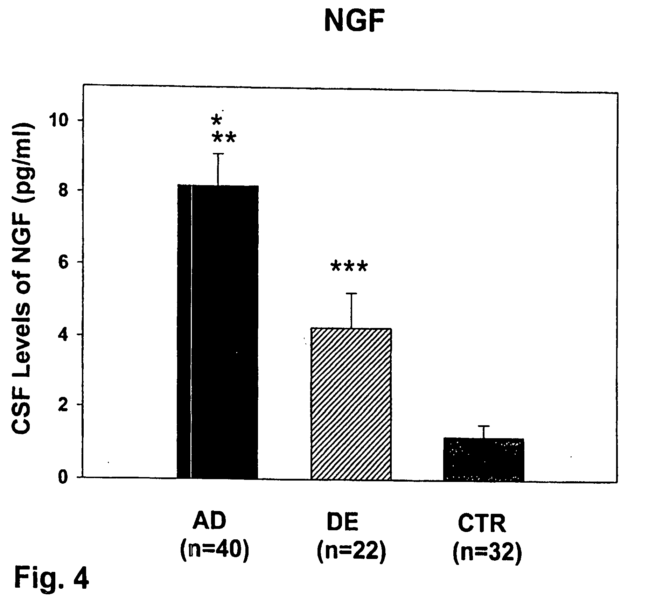

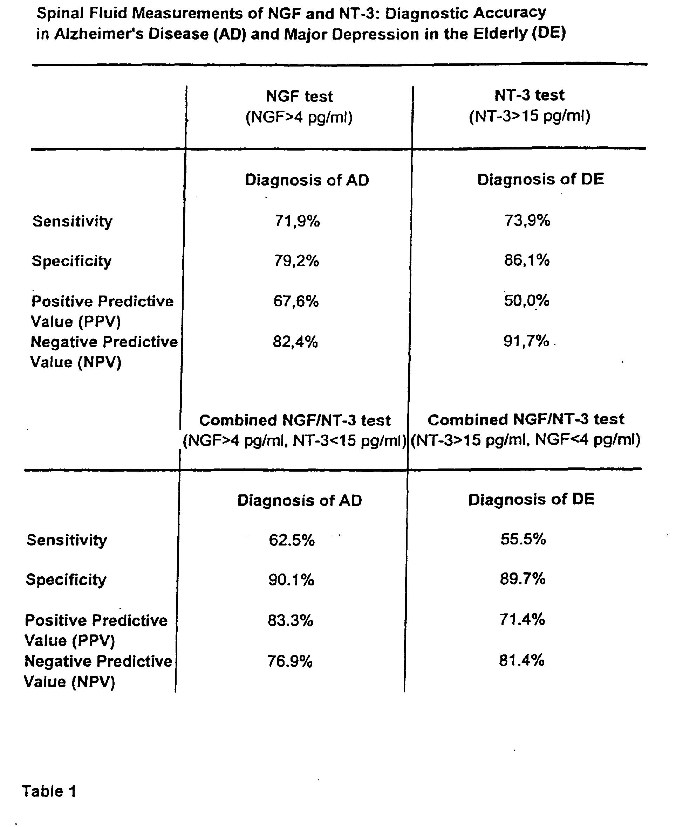

Figure 4 relates to example 3 and depicts nerve growth factor (NGF) levels in the cerebrospinal fluid of patients with Alzheimer's disease (AD), major depression in the elderly (DE) and non-demented control subjects. CSF levels of NGF were determined to define a cut-off value to be used in the combined tests shown in table 1. Levels (pg/ml) are given in mean ± SEM. Asterix (*, **, ***) indicate significance (p < 0.05), Mann-Whitney U test. * AD versus DE, p = 0.002; ** AD versus CTR, p = 0.000, *** DE versus CTR, p = 0.000. CSF levels of NGF were significantly elevated in the AD group, as compared to both the DE and the CTR group. CSF levels of NGF were also significantly elevated in the DE group, as compared to the CTR group. NGF concentrations in CSF of the AD group amounted to 8.19 +/- 0.91 pg/ml (mean +/- SEM, range: 0.00 to 23.00, n = 40), compared to 4.26 +/- 0.97 pg/ml in the DE group (range: 0.00 to 23.00, n = 22), and 1.18 +/- 0.35 pg/ml in the CTR group (range: 0.00 to 7.20, n = 32), respectively.

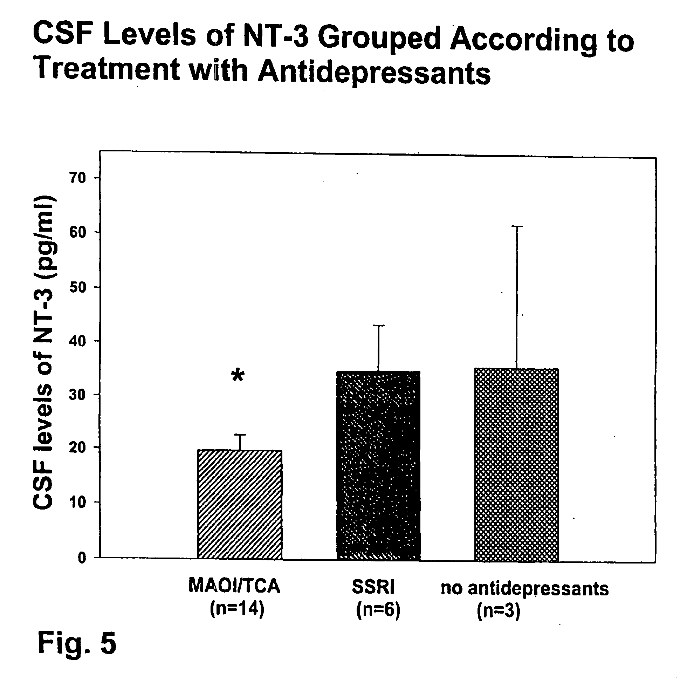

Figure 5 depicts CSF levels of NT-3 in DE patients (n = 23) grouped according to treatment with antidepressants: substances that affect central noradrenergic neurotransmission (MAOI and TCA, n = 14); substances that selectively affect serotonergic neurotransmission (SSRI, n =6); no treatment with antidepressants at the time of lumbar puncture (N=3). CSF levels of NT-3 (pg/ml) are given in mean ± SEM. Asterix (*) indicates significance (p < 0.05, independent samples t - test, MAOI/TCA versus SSRI). Interestingly, Smith et al. (Proc. Natl. Acad. Sci. USA, 92 (19), 8788 - 8792, 1995) showed that chronic treatment with antidepressants that selectively blocked neuronal noradrenaline uptake decreased the expression levels of NT-3 in the locus coeruleus in rats. In contrast, treatment with selective serotonin reuptake inhibitors did not alter NT-3 mRNA levels. These findings suggest that some effects of antidepressants on the function of the locus coeruleus involve NT-3 expression. However, these findings make it highly unlikely that the increased CSF levels of NT-3 in patients with DE are due to effects of treatment with antidepressants. In fact, CSF levels of NT-3 were markedly lower within the DE group in patients treated with substances that affect central noradrenergic neurotransmission (MAOI/TCA) than in patients treated with substances that selectively affect serotonergic neurotransmission (SSRI) as well as in patients that were not treated with antidepressants at the time of lumbar puncture. Thus, treatment with antidepressants that block neuronal noradrenaline uptake may reduce previously increased CSF levels of NT-3 in patients with DE.

Table 1 relates to examples 2 and 3. This table shows the diagnostic accuracy of spinal fluid measurements of NT-3 and NGF in Alzheimer's Disease and Major Depression in the elderly. In NT-3 measurements, sensitivity, specificity, positive predictive value (PPV) and negative predictive value (NPV) were calculated using a cut-off value of ≥ 15.0 pg/ml (total: n = 125, AD: n = 39, DE: n = 23, CTR: n = 63). For combined NT-3/NGF measurements in the diagnosis of DE, cut-off values of < 4 pg/ml NGF, and ≥ 15 pg/ml NT-3, respectively (total: n = 57, AD: n = 24, DE: n = 18, CTR: n = 15) were used. CSF measurement of NT-3 levels showed a promising diagnostic accuracy in terms of sensitivity (73.9 %) as well as specificity (86.1 %). Thus, the NT-3 test constitutes a candidate tool for the biochemical diagnosis of major depression in the elderly. In the combined test for DE diagnosis, the specificity is slightly increased (89.7 %). For combined NT-3/NGF measurements in the diagnosis of AD, cut-off values of < 15 pg/ml NT-3 and ≥ 4 % pg/ml NGF, respectively (total n = 57; AD n = 24, DE n = 18, CTR n = 15) were used. This combined test shows a considerable specificity for the diagnosis of AD (90.1 %). In general, combined testing of NT-3 and NGF with suitable cut-off values has the potential to be used in the biochemical differentiation between these two frequent disorders in the elderly.

### EXAMPLE 1

In order to achieve a differential diagnosis, the study included not only patients with major depression (DE), but also such with Alzheimer's disease. Patients with major depression were diagnosed according to the ICD10 (F32.0x/1x, F33.0x/1x) and DSM-IIIR (296.20-22, 296.30-32) criteria. Diagnosis of probable AD was made according to criteria of the National Institute of Neuropsychiatric and Communicative Disorders and Stroke-Alzheimer's disease and Related Disorders Association (NINCDS-ADRDA; McKhann et al., Neurology 34, 939 - 944, 1984). All patients were referred to the research ward from general practitioners, neurologists and psychiatrists for diagnostic purposes and screening for clinical trials. None of the patients was institutionalized. The group of healthy control subjects (CTR) consisted of patients that underwent lumbar puncture for orthopedic or neurologic diagnostic purposes and were shown to have normal CSF cell counts, total protein levels, and absence of signs of blood barrier dysfunction or cerebral IgG synthesis, as well as absence of any cerebral disorders.

DE, AD and CTR patients were carefully examined and received a thorough clinical work-up. Psychometric testing including the Mini Mental State (MMS; Folstein et al., J. Psychiatry Res. 12, 189 - 198, 1975), as a global screening instrument for dementia, and the Nurses' Observation Scale for Geriatric Patients (NOSGER; Spiegel et al., J. Am. Geriatr. Soc. 39(4), 339 - 347, 1991) as a functional measure of dementia severity. The patients with DE showed no cognitive disturbances in the clinical examinations and the Mini Mental State scores were within the normal range. Severity of depression was rated by using the Montgomery Asberg Depression Rating Scale (MADRS) (Montgomery et al., Br. J. Psychiatry, 134: 382 - 389, 1979). Apolipoprotein (ApoE) genotyping, or, if DNA was not available, ApoE phenotyping was included in the laboratory screening in the DE and AD patients.

CSF was obtained for diagnostic purposes in the DE and AD patients in which no lumbar puncture had been previously done during the routine diagnostic work-up. Different CSF volumes were available for the analysis of the neurotrophin proteins. This fact explains the different sample sizes for the individual measurements. All available CSF samples were used for the analyses.

The AD group was as follows: n = 23, 12 men, 11 women, mean age 63.9 +/-13.2 SD years, range 39 - 86 years, MMS score: mean 18.6 +/- 5.6 SD.

The DE group was as follows: n = 14, 5 men, 9 women, mean age 68.2 +/-13.6 SD years, range 47 - 86 years, MMS score: mean 28.1 +/- 0.9 SD.

The CTR group was as follows: n = 14, 8 men, 6 women, mean age 58.5 +/-16.2 SD years, range 31 - 81 years.

AD and CTR patients were free of psychotropic medication. Patients with major depression were treated with various antidepressant drugs including serotonin reuptake inhibitors, reversible monoaminooxidase A inhibitors and tricyclics. Informed consent was taken from each patient and their caregivers before the investigation. The study was approved by the local ethics committee. All procedures were in accordance with the Helsinki Declaration of 1975, as revised in 1983. Within one week of dementia testing, CSF was obtained by lumbar puncture. To control for possible influences of a ventriculo-lumbar gradient, lumbar punctures were done between 7.30 and 8 a. m. before breakfast while patients were still lying flat. CSF samples were frozen on dry ice immediately upon withdrawal at the bedside in 0.5 ml aliquots and stored at - 85 °C until biochemical analysis.

NT-3 was determined using commercially available ELISA systems (Promega, Madison, WI). The determination was performed according to the manufacture's protocol. 220 µl of undiluted CSF in carbonate buffer (pH 9.7) were added to 96 well immunoplates (Nunc) at 4 °C overnight. Anti-Human-NT-3 polyclonal antibodies (pAb) were used as capture antibody. Anti-NT-3 monoclonal antibody (mAb) were used as reporter antibody. After incubation with a species-specific Ab (anti-rat IgG) conjugated to horseradish peroxidase (HRP) as a tertiary reactant, and washing, the solution was incubated with the chromogenic substrate TMB (3, 5, 3', 5'-tetramethylbenzidine). Absorbance was measured at 450 nm using a microplate reader (Dynatech MR 700). NT-3 ELISA: range 4.7 - 300 pg/ml; cross-reaction with other neurotrophins at 10 ng/ml < 3 %; detection limit 10.0 pg/ml. All CSF samples were assayed in duplicate determinations.

ApoE genotyping was performed using INNO-LiPA ApoE, Innogenetics, Belgium. ApoE phenotyping was performed according to McDowell et al. (Clin. Chem. 35(10), 2070 - 2073, 1989). The use of the ApoE phenotype synonymous with the ApoE genotype in the statistical analyses seemed to be appropriate, since ApoE genotyping compared with protein phenotyping showed conflicting results in less than 2 % (Hansen et al., Clin. Chim. Acta, 224(2), 131 - 137, 1994).

Statistical analyses of data were performed using the Mann-Whitney U test as well as independent samples *t*-tests for group comparisons. Correlation analyses were performed by multiple regression using CSF levels of neurotrophins as well as ApoE genotype (or phenotype, respectively), age, duration of the disease in AD, MMS, NOSGER and MADRS scores. Non-parametric correlation was performed using the Kruskal-Wallis test. Regression analysis was complemented with analysis of variance (ANOVA) by using SPSS for Windows (version 8.0). Statistical significance was assumed at p < 0.05. Bonferroni correction for multiple testing was applied.

### EXAMPLE 2

The study described in example 1 has been extended to a wider panel of patients as described below in this example 2.

Diagnosis, clinical examination and treatment of patients as well as lumbar puncture were performed as described in example 1.

For NT-3 measurements, 125 spinal fluid samples were examined. DE group: n = 23, 8 men, 15 women, mean age 70.5 +/- 11.9 SD years, range 47 - 86 yr, MMS score: mean 27.2 +/- 2.5 SD. AD group: n = 39, 20 men, 19 women, mean age 67.2 +/- 11.5 SD years, range 39 - 86 yr, MMS score: mean 19.1 +/- 5.3 SD. CTR group: n = 63, 35 men, 28 women, mean age 56.0 +/- 15.0 SD years, range 28 - 84 yr.

NT-3 was determined by using commercially available ELISA systems (Promega, Madison, WI) according to the manufacturer's protocol. 120 µl of undiluted CSF in carbonate buffer (pH 9.7) were added to 96 well immunoplates (Nunc) at 4 °C overnight. Anti-Human-NT-3 polyclonal antibodies (pAb) were used as capture Ab. Anti-NT-3 mAb were used as reporter Ab. After incubation with a species-specific Ab (anti-rat IgG) conjugated to horseradish peroxidase (HRP) as a tertiary reactant, and washing, the solution was incubated with the chromogenic substrate TMB. Absorbance was measured at 450 nm by using a microplate reader (Dynatech MR 700). NT-3 ELISA: linear range 4.7 - 300 pg/ml; cross-reaction with other neurotrophins at 10 ng/ml < 3%; detection limit 6.0 pg/ml.

AD and CTR patients were free of psychotropic medication. Patients with major depression were treated with various antidepressants: 11 were treated with tricyclics (TCA), 2 with a monoamine oxidase-A inhibitor (MAOI), 1 with a combination of TCA with MAOI, 6 with selective serotonin reuptake inhibitors (SSRI), and 3 were free of antidepressants at the time of lumbar puncture.

Statistical analyses of data were performed using the Mann-Whitney U test for group comparisons. Regression analysis was complemented with analysis of variance (ANOVA) by using SPSS for Windows (version 8.0). Statistical significance was assumed at p < 0.05. Bonferroni correction for multiple testing was applied.

To estimate the diagnostic accuracy of the test, a) sensitivities and b) specificities, defined as follows, were calculated: a) true positives / (true positives and false negatives), and b) true negatives / (true negatives and false positives). To estimate the probability of disease, predictive values of the tests were calculated. The positive predictive value (PPV) was defined as true positives / (true positives + false positives). The negative predictive value (NPV) was defined as true negatives / (true negatives + false negatives).

Next, DE patients (n = 23) were grouped according to treatment with substances that affect central noradrenergic neurotransmission (MAOI and TCA, n = 14) and those that selectively affect serotonergic neurotransmission (SSRI, n = 6). CSF levels of NT-3 were significantly lower in the MAOI/TCA group, as compared to the SSRI group (p < 0.05, independent samples t - test). NT-3 concentrations in CSF of the MAOI/TCA group were 19.7 ± 2.9 pg/ml (mean ± SEM, range: 0.0 to 35.0, n = 14), compared to 34.9 ± 8.5 pg/ml in the SSR group (range: 14.0 to 63.0, n = 6), and 35.7 ± 26.3 pg/ml in the group with patients that were not treated with antidepressants at the time of lumbar puncture (range: 0.00 to 87.0, n = 3) (Fig. 5).

### EXAMPLE 3

The purpose of this study was to check whether combined measurements of the CSF levels of NT-3 and nerve growth factor (NGF) - which also belongs to the group of neurotrophins - improves the diagnostic accuracy of the NT-3 test described in example 2.

In a first step, NGF levels in CSF were determined to define a cut-off value to be used in the combined tests. Diagnosis, clinical examination and treatment of patients, lumbar puncture and statistical analyses were performed as described in example 2. 94 spinal fluid samples were examined. The AD group (n= 40) consisted of 18 men and 22 women, mean age 68.8 +/- 12.4 years, range 39 - 88 yr, MMS score: mean 19.3 +/- 4.6 SD. The DE group (n = 22) consisted of 8 men and 12 women, mean age 69.8 +/- 12.6 SD years, range 47 - 86 yr, MMS score: mean 27.5 +/- 2.1 SD. CTR group: n = 32, 18 men, 14 women, mean age 64.0 +/- 14.9 SD years, range 29 - 96 yr. CSF levels of NGF were measured by an ELISA as described by Weskamp et al. (J. Neurochem. 48: 1779 - 1786, 1987). Black 96-well microplates (Nunc) were coated with monoclonal anti-β (2.5 S, 7S) NGF antibodies (Ab) (clone 27/21, Boehringer Mannheim) diluted in carbonate buffer pH 9.2 overnight at 4 °C. 120 µl of CSF and standard solutions were added and incubated for 20 hours at 4 °C. Plates were washed and incubated with anti-β (2.5 S, 7S) NGF-β-galactosidase conjugate for 2 ½ hours at room temperature (RT). Following an additional washing step, the fluorogenic substrate 4-methylumbelliferyl-β-D-galactopyranoside was added and plates were incubated at 4 °C overnight. The reaction was stopped after 1h at RT, and the fluorescent product was measured in the microtiter wells by using a fluorometer (Labsystems Fluoroskan Ascent FL) at 355 nm excitation and 460 nm emission wavelength. The detection limit was 0.5 pg/ml; the cross-reactivity with other neurotrophins at 10 ng/ml was < 2 % and the assay was linear over a range of 0.5 to 500 pg/ml.

57 CSF samples were available for combined NGF/NT=3 measurements. AD group: n = 24, 13 men, 11 women, mean age 64.9 +/- 12.5 SD years, range 47 - 82 yr, MMS score: mean 18.6 +/- 5.4 SD. DE group: n = 18, 7 men, 11 women, mean age 69.5 +/- 12.7 SD years, range 47 -84 yr, MMS score: mean 27.7 +/- 2.1 SD. CTR group: n = 15, 10 men, 5 women, mean age 59.0 +/- 15.9 SD years, range 29 - 80 yr.

## Claims

1. An in vitro method of diagnosing or prognosing depression, in particular major depression or determining an increased risk of developing depression, in particular major depression, comprising:
determining a level, or an activity, or both said level and said activity, of neurotrophin 3 and/or of,a transcription product of a gene coding for neurotrophin 3 in a cerebrospinal fluid sample from a human being;
and comparing said level, or said activity, or both said level and said activity, to a reference value representing a known disease or health status,
wherein an increase of said level of neurotrophin 3 in said cerebrospinal fluid from said human being relative to said reference value representing a known health status indicates a diagnosis, or prognosis, or increased risk of depression, in particular major depression, in said human being,
thereby diagnosing or prognosing depression in said human being, or determining whether said human being is at increased risk of developing depression.

2. An in vitro method of monitoring the progression of depression, in particular major depression, comprising:
determining a level, or an activity, or both said level and said activity, of neurotrophin 3 and/or of a transcription product of a gene coding for neurotrophin 3 in a cerebrospinal fluid sample from a human being;
and comparing said level, or said activity, or both said level and said activity, to a reference value representing a known disease or health status,
wherein an increase of said level of neurotrophin 3 in said cerebrospinal fluid from said human being relative to said reference value representing a known health status indicates a diagnosis, or prognosis, or increased risk of depression, in particular major depression, in said human being,
thereby monitoring the progression of depression in said human being.

3. An in vitro method of evaluating a treatment for depression, in particular major depression, comprising:
determining a level, or an activity, or both said level and said activity, of neurotrophin 3 and/or of a transcription product of a gene coding for neurotrophin 3 in a cerebrospinal fluid sample from a human being;
and comparing said level, or said activity, or both said level and said activity, to a reference value representing a known disease or health status,
wherein an increase of said level of neurotrophin 3 in said cerebrospinal fluid from said human being relative to said reference value representing a known health status indicates a diagnosis, or prognosis, or increased risk of depression, in particular major depression, in said human being,
thereby evaluating said treatment for depression.

4. The method according to claim 1, wherein a level of neurotrophin 3 ≥ 15 µg/ml in said cerebrospinal fluid indicates a diagnosis, or prognosis, or increased risk of depression, in particular major depression, in said human being.

5. The method according to claim 4, wherein a level of neurotrophin 3 in the range from 15 pg/ml to 50 pg/ml in said cerebrospinal fluid indicates a diagnosis, or prognosis, or increased risk of depression, in particular major depression, in said human being.

6. The method according to any of claims 1 to 5, wherein neurotrophin 3 and/or said transcription product is detected using an immunoassay and/or a binding assay.

7. The method according to any of claims 1 to 6, further comprising comparing a level, or an activity, or both said level and said activity, of neurotrophin 3 and/or of a transcription product of a gene coding for neurotrophin 3 in said cerebrospinal fluid sample with a level, or an activity, or both said level and said activity, of at least one of said substances in a series of cerebrospinal fluid samples taken from said human being over a period of time.

8. The method according to claim 7, wherein said human being receives a treatment prior to one or more of said cerebrospinal fluid sample gatherings.

9. The method according to claim 8, wherein said level, or said activity, or both said level and said activity, in said cerebrospinal fluid sample is determined, before and after said treatment of said human being.

10. Use of a kit comprising at least one reagent which selectively detects neurotrophin 3 or a transcription product of a gene coding for neurotrophin 3 for the in vitro diagnosis, or prognosis, or determination of increased risk of developing depression, in particular major depression.

## Patentansprüche

1. In-vitro-Verfahren zum Diagnostizieren oder Prognostizieren einer Depression, insbesondere einer Major Depression (Typischen Depression), oder zum Bestimmen eines erhöhten Risikos, eine Depression, insbesondere eine Major Depression, zu entwickeln, umfassend:
Bestimmen einer Konzentration oder einer Aktivität oder sowohl einer Konzentration als auch einer Aktivität von Neurotrophin 3 und/oder eines Transcriptionsprodukts eines Gens, das für Neurotrophin 3 codiert, in einer Liquorprobe von einem Menschen; und
Vergleichen der Konzentration oder der Aktivität oder sowohl der Konzentration als auch der Aktivität mit einem Referenzwert, der einen bekannten Krankheits- oder Gesundheitszustand darstellt;
wobei eine Erhöhung der Konzentration des Neurotrophin 3 im Liquor des Menschen relativ zu dem Referenzwert, der einen bekannten Gesundheitszustand darstellt, eine Diagnose oder Prognose oder ein erhöhtes Risiko einer Depression, insbesondere Major Depression, bei dem Menschen anzeigt;
wodurch eine Depression bei dem Menschen diagnostiziert oder prognostiziert wird oder bestimmt wird, ob der Mensch ein erhöhtes Risiko hat, eine Depression zu entwickeln.

2. In-vitro-Verfahren zur Überwachung des Fortschritts einer Depression, insbesondere einer Major Depression, umfassend:
Bestimmen einer Konzentration oder einer Aktivität oder sowohl einer Konzentration als auch einer Aktivität von Neurotrophin 3 und/oder eines Transcriptionsprodukts eines Gens, das für Neurotrophin 3 codiert, in einer Liquorprobe von einem Menschen; und
Vergleichen der Konzentration oder der Aktivität oder sowohl der Konzentration als auch der Aktivität mit einem Referenzwert, der einen bekannten Krankheits- oder Gesundheitszustand darstellt;
wobei eine Erhöhung der Konzentration des Neurotrophin 3 im Liquor des Menschen relativ zu dem Referenzwert, der einen bekannten Gesundheitszustand darstellt, eine Diagnose oder Prognose oder ein erhöhtes Risiko einer Depression, insbesondere Major Depression, bei dem Menschen anzeigt;
wodurch der Fortschritt der Depression bei dem Menschen überwacht wird.

3. In-vitro-Verfahren zur Bewertung einer Behandlung einer Depression, insbesondere einer Major Depression, umfassend:
Bestimmen einer Konzentration oder einer Aktivität oder sowohl einer Konzentration als auch einer Aktivität von Neurotrophin 3 und/oder eines Transcriptionsprodukts eines Gens, das für Neurotrophin 3 codiert, in einer Liquorprobe von einem Menschen; und
Vergleichen der Konzentration oder der Aktivität oder sowohl der Konzentration als auch der Aktivität mit einem Referenzwert, der einen bekannten Krankheits- oder Gesundheitszustand darstellt;
wobei eine Erhöhung der Konzentration des Neurotrophin 3 im Liquor des Menschen relativ zu dem Referenzwert, der einen bekannten Gesundheitszustand darstellt, eine Diagnose oder Prognose oder ein erhöhtes Risiko einer Depression, insbesondere Major Depression, bei dem Menschen anzeigt;
wodurch die Behandlung der Depression bewertet wird.

4. Verfahren gemäß Anspruch 1, wobei eine Konzentration an Neurotrophin 3 von ≥ 15 pg/ml in dem Liquor eine Diagnose oder Prognose oder ein erhöhtes Risiko einer Depression, insbesondere Major Depression, bei dem Menschen anzeigt.

5. Verfahren gemäß Anspruch 4, wobei eine Konzentration an Neurotrophin 3 im Bereich von 15 pg/ml bis 50 pg/ml in dem Liquor eine Diagnose oder Prognose oder ein erhöhtes Risiko einer Depression, insbesondere Major Depression, bei dem Menschen anzeigt.

6. Verfahren gemäß einem der Ansprüche 1 bis 5, wobei das Neurotrophin 3 und/oder das Transcriptionsprodukt mit Hilfe eines Immunoassays und/oder eines Bindungsassays nachgewiesen wird.

7. Verfahren gemäß einem der Ansprüche 1 bis 6, das weiterhin Folgendes umfasst: Vergleichen einer Konzentration oder einer Aktivität oder sowohl einer Konzentration als auch einer Aktivität von Neurotrophin 3 und/oder eines Transcriptionsprodukts eines Gens, das für Neurotrophin 3 codiert, in der Liquorprobe mit einer Konzentration oder einer Aktivität oder sowohl einer Konzentration als auch einer Aktivität von wenigstens einer der Substanzen in einer Reihe von Liquorproben, die über einen Zeitraum hinweg von dem Menschen entnommen wurden.

8. Verfahren gemäß Anspruch 7, wobei der Mensch vor einer oder mehreren der Entnahmen der Liquorprobe eine Behandlung erhält.

9. Verfahren gemäß Anspruch 8, wobei die Konzentration oder die Aktivität oder sowohl die Konzentration als auch die Aktivität in der Liquorprobe vor und nach der Behandlung des Menschen bestimmt wird.

10. Verwendung eines Kits, der wenigstens ein Reagens umfasst, das selektiv Neurotrophin 3 oder ein Transcriptionsprodukt eines Gens, das für Neurotrophin 3 codiert, nachweist, für die In-vitro-Diagnose oder -Prognose einer Depression, insbesondere einer Major Depression, oder für die In-vitro-Bestimmung eines erhöhten Risikos, eine solche zu entwickeln.

## Revendications

1. Procédé *in vitro* permettant de diagnostiquer ou de pronostiquer une dépression, notamment une dépression importante ou de déterminer un risque majoré de développer une dépression, notamment une dépression importante, ledit procédé comportant :
la détermination d'un taux, ou d'une activité, ou à la fois dudit taux et de ladite activité, de la neurotrophine 3 et/ou d'un produit de transcription d'un gène codant pour la neurotrophine 3 dans un fluide cérébrospinal corporel d'un être humain ;
et la comparaison dudit taux, ou de ladite activité, ou à la fois dudit taux et de ladite activité, avec une valeur de référence représentant une maladie connue ou un état de santé connu,
dans lequel une augmentation dudit taux de la neurotrophine 3 dans ledit fluide corporel prélevé sur ledit être humain par rapport à ladite valeur de référence représentant un état de santé connu indique un diagnostic, ou pronostic, ou un risque majoré de dépression, notamment de dépression importante, chez ledit être humain,
ce qui permet de diagnostiquer ou pronostiquer une dépression chez ledit être humain, ou de déterminer si ledit être humain présente un risque majoré de développer une dépression.

2. Procédé *in vitro* permettant de surveiller la progression d'une dépression, notamment d'une dépression importante, ledit procédé comportant :
la détermination d'un taux, ou d'une activité, ou à la fois dudit taux et de ladite activité, de la neurotrophine 3 et/ou d'un produit de transcription d'un gène codant la neurotrophine 3 dans un fluide cérébrospinal corporel d'un être humain ;
et la comparaison dudit taux, ou de ladite activité, ou à la fois dudit taux et de ladite activité, avec une valeur de référence représentant une maladie connue ou un état de santé connu,
dans lequel une augmentation dudit taux de la neurotrophine 3 dans ledit fluide corporel prélevé sur ledit être humain par rapport à ladite valeur de référence représentant un état de santé connu indique un diagnostic, ou un pronostic, ou un risque majoré de dépression, notamment de dépression importante, chez ledit être humain, ce qui permet de surveiller la progression de la dépression chez ledit être humain.

3. Procédé *in vitro* permettant d'évaluer un traitement d'une dépression, notamment d'une dépression importante, ledit procédé comportant :
la détermination d'un taux, ou d'une activité, ou à la fois dudit taux et de ladite activité, de la neurotrophine 3 et/ou d'un produit de transcription d'un gène codant la neurotrophine 3 dans un fluide cérébrospinal corporel d'un être humain ;
et la comparaison dudit taux, ou de ladite activité, ou à la fois dudit taux et de ladite activité, avec une valeur de référence représentant une maladie connue ou un état de santé connu,
dans lequel une augmentation dudit taux de la neurotrophine 3 dans ledit fluide corporel prélevé sur ledit être humain par rapport à ladite valeur de référence représentant un état de santé connu indique un diagnostic, ou un pronostic, ou un risque majoré de dépression, notamment de dépression importante, chez ledit être humain, ce qui permet d'évaluer ledit traitement de la dépression.

4. Procédé selon la revendication 1, dans lequel un niveau de de neurotrophine 3 supérieur ou égal à 15 pg/ml dans ledit fluide corporel indique un diagnostic, ou un pronostic, ou un risque majoré de dépression, notamment de dépression importante, chez ledit être humain.

5. Procédé selon la revendication 4, dans lequel un niveau de neurotrophine 3 dans la gamme de 15 pg/ml à 50 pg/ml dans ledit fluide corporel indique un diagnostic, ou un pronostic, ou un risque majoré de dépression, notamment de dépression importante, chez ledit être humain.

6. Procédé selon l'une quelconque des revendications 1 à 5, dans lequel la neurotrophine 3 et/ou ledit produit de transcription est détecté en utilisant une essai immunologique et/ou un essai de liaison.

7. Procédé selon l'une quelconque des revendications 1 à 6, comportant en outre la comparaison d'un niveau, ou d'une activité, ou à la fois ledit niveau et ladite activité, de la neurotrophine 3 et/ou d'un produit de transcription d'un gène codant la neurotrophine 3 dans ledit fluide cérébrospinal corporel avec un taux, ou une activité, ou à la fois ledit niveau et ladite activité, d'au moins une desdites substances dans une série de fluides cérébrospinaux corporels prélevés sur ledit être humain pendant un intervalle de temps.

8. Procédé selon la revendication 7, dans lequel ledit être humain reçoit un traitement avant un ou plusieurs prélèvements desdits fluides cérébrospinaux corporels.

9. Procédé selon la revendication 8, dans lequel ledit niveau, ou ladite activité, ou à la fois ledit niveau et ladite activité, dans ledit fluide cérébrospinal corporel est déterminé, avant et après ledit traitement dudit être humain.

10. Utilisation d'un kit comprenant au moins un réactif qui détecte sélectivement la neurotrophine 3 ou un produit de transcription d'un gène codant la neurotrophine 3 pour le diagnostic *in vitro,* ou le pronostic, ou la détermination d'un risque majoré de développer une dépression, notamment une dépression importante.
